# EUROPEAN PATENT SPECIFICATION

(11) **EP 2 602 003 B1**
(45) Date of publication and mention of the grant of the patent: **16.07.2014**
(21) Application number: 11192198.7
(22) Date of filing: 06.12.2011
(51) Int. Cl.: A61N 5/10, G21K 5/02, G21K 5/04

(54) **Combined ion irradiation and ion radiography device**
Kombinierte Ionenbestrahlungs- und Ionenradiographievorrichtung
Dispositif combiné de radiographie et d'irradiation ionique

(43) Date of publication of application: 12.06.2013
(73) Proprietor: GSI Helmholtzzentrum für Schwerionenforschung GmbH, 64291 Darmstadt (DE)
(72) Inventor: Durante, Marco, 64287 Darmstadt (DE); Stöcker, Horst, 61440 Oberursel (DE)

(56) References cited:
- EP-A1- 2 400 506
- WO-A1-2006/094533
- SCHNEIDER U ET AL: "PROTON RADIOGRAPHY AS A TOOL FOR QUALITY CONTROL IN PROTON THERAPY", MEDICAL PHYSICS, AIP, MELVILLE, NY, US, vol. 22, no. 4, 1 April 1995 (1995-04-01), pages 353-363, XP000511072, ISSN: 0094-2405, DOI: 10.1118/1.597470

## Description

The invention relates to an irradiation arrangement for irradiating a workpiece with a particle beam, comprising at least one particle beam supply device, at least one workpiece accommodation device and at least one position-sensitive particle monitoring device. The invention further relates to a method for controlling an irradiation arrangement for irradiating a workpiece with a particle beam, wherein a particle beam is used that is penetrating the workpiece to be irradiated.

The irradiation of a workpiece with a particle beam is nowadays a standard method in science, industry and even in the medical sector. Depending on the technical field, where an irradiation of a workpiece is employed, the workpiece can be taken from a huge variety of possibilities. For example, in industry it can be an industrial product, like a piece of machinery, an electronic integrated circuit (for example a micro processor, an electronic memory module etc.) and in the medical sector it can be a radiation phantom for testing an irradiation scheme, an animal or even a human patient. Furthermore, the types of particles, the way how the radiation is applied (for example using a thin, pencil-like beam or a huge homogenous area to be irradiated) and the particle energy can be chosen from a wide variety. Just to name a few examples: sometimes infrared light is used for heating up a workpiece. Due to the characteristics of infrared light, the photons (which are particles, although they have no rest mass) are usually not penetrating the workpiece to be treated. On the other hand, sometimes x-rays are used (which are photons with a high energy), where usually a substantial fraction of the x-rays penetrates the workpiece. This way, an object can be inspected and/or it is possible to use the radiation that is deposited within the workpiece to alter at least a part of the workpiece.

However, not only photons are used for irradiating workpieces. Instead, also particles, showing a rest mass, are used for irradiating workpieces.

As an example, electrons are widely used for a variety of purposes. Just to name an example, an energetic electron beam is frequently used in industry for soldering or welding two pieces together. In medicine, electron beams are sometimes used for treating cancer or a variety of other diseases.

Even more, nowadays highly energetic proton beams are not only used in research (for irradiating a target or a workpiece for investigating inner structures of matter or investigating properties of a material in material science), but also in other fields like industry and medicine. Protons have the advantage that they show a so-called Bragg-peak. That is, when penetrating material, protons do not lose their energy along the penetration path linearly. Instead, they lose a comparatively small energy in the beginning. Only shortly before they finally get stopped in the matter, they lose the majority of their energy. This way, a three dimensional structuring can be performed within a workpiece on the millimeter scale, even when only one particle path is used. This Bragg-peak is not unique to protons, but it also occurs with other types of hadrons. In particular heavy ions (like helium ions, carbon ions, nitrogen ions or neon ions) show a profound Bragg-peak as well. Usually, the Bragg-peak becomes even more pronounced, when the rest mass of the particles used is increased.

Due to this unique characteristic of a Bragg-peak, heavy ions have been introduced some years ago for the treatment of cancer, in particular in cases where the tumor is in a place where it cannot be reached by surgery. Different methods on how to apply the heavy ion particle beam to a certain, defined volume to be irradiated with a certain dose have been suggested in the state of the art.

A certain problem arises when workpieces have to be treated that are not identical to each other. Such a problem can occur in all types of technical fields. However, for medical applications this problem is essentially always present, since every patient differs and their diseases are usually unique to the respective patient. If such a situation occurs, it is usually necessary to inspect the workpiece to be treated before the irradiation may begin. However, the desire to inspect the workpiece to be treated (for example the location and size of a special volume to be irradiated within a workpiece) can be present even during the irradiation process. This is particularly the case, if the workpieces (or parts of the workpiece) is moving. In medicine, this can occur due to breathing, peristaltic movements of the intestines or the heartbeat.

When heavy ions are used for irradiating a workpiece, according to the state of the art a second measuring method for determining the (inner) movement of the workpiece to be treated is usually employed. For example, a workpiece is irradiated with a heavy ion beam, while the (inner) the movement of the workpiece is measured with x-rays, computer tomography, movement substitutes (like an expandable belt that is placed around the chest of a patient) or the like. Although such imaging techniques work in principle already, they show some disadvantages as well. In particular, if different types of particles are used for irradiating and monitoring (or even a completely different measurement method is employed for monitoring), the monitoring does not take into account the special interaction properties between the irradiation particles and the matter to be irradiated. This can be problematic in some applications.

So far, no heavy research has been performed to reduce errors that are induced by this problem.

Therefore, there is a need for an improved irradiation arrangement for irradiating a workpiece with a particle beam, as well as for an improved method for controlling an irradiation arrangement for irradiating a workpiece to be irradiated.

The invention solves the problem.

Is it is suggested to design an irradiation arrangement for irradiating a workpiece with a particle beam, wherein the irradiation arrangement comprises at least one particle beam supply device, at least one workpiece accommodation device and at least one position-sensitive particle monitoring device, in a way that said at least one position-sensitive particle monitoring device is arranged at least in part on the distal side of said at least one workpiece accommodation device, when seen from said at least one particle beam supply device. This way, it is suggested to "use the particle beam twice", namely (at least) for irradiating at least one workpiece that is arranged on the at least one workpiece accommodation device and furthermore for monitoring the outgoing particle beam, i.e. the particle beam after having passed the at least one workpiece. It is presently proposed to monitor the particle beam with respect to a position of the particle beam. Usually, this position is measured in an x-y-plane (where the z-direction is the direction of the particle beam). Although an orthogonal coordinate system is preferably used (at least for the x- and the y-axis), other angles are possible as well. It is to be noted that although the use of position-sensitive particle monitoring devices is known in connection with particle beams, so far the position-sensitive particle monitoring devices have been usually placed in front of (proximal to) the workpiece accommodation device. This makes perfect sense, since at this position, the particle beam is yet undisturbed. It has been previously believed that positioning the position-sensitive particle monitoring device on the distal side of the at least one workpiece accommodation device, will cause unavoidable, unnecessary and undesired deviations of the particle beam. This is due to scattering events and reemission events (to name some examples) within the workpiece. It should be mentioned that the particle beam that is used for irradiating the workpiece (i.e. in general the particle beam that is leaving the at least one particle beam supply device) and the particle beam that is used for measurement is the very same. Surprisingly, the inventors have figured out that using the "already disturbed" particle beam is particularly well-suited for monitoring the workpiece that is currently irradiated. This is particularly the case, since the physical interaction between the particles used both for monitoring and irradiation are the same, since the particles are necessarily of the same type, energy and/or charge etc. (since they come from the same particle beam). Usually, a clear distinction between a "pure" radiographic irradiation and a "combined" therapeutic and radiographic irradiation can be made by the luminosity of the irradiation and/or the dose that is deposited in the workpiece to be treated. The at least one particle beam supply device can be essentially of any type. For example, it can be a window-like opening where one side can be kept under vacuum while the other side is under standard atmospheric conditions. Such a type of opening can have both a window or no window. Of course, it is also possible that the whole irradiation arrangement (or at least one, two or all three) made out of the particle beam supply device, the workpiece accommodation device and the position sensitive particle monitoring device is operated under vacuum. Of course, the particle beam supply device can comprise even more components like bending magnets, a gantry, focusing devices or can even be a whole particle accelerator unit. The workpiece accommodation device can be essentially any type of device where a workpiece can be placed. Preferably, the workpiece can be fixed, can be positioned to a desired position and/or can be moved. The position sensitive particle monitoring device can be designed as a wire chamber, as capacitor like plates, as charged couple devices or any other position sensitive device that is known in the state of the art or will be developed in the future.

Preferably the irradiation arrangement is designed in a way that said at least one position-sensitive particle monitoring device is designed, at least in part, as an imaging device. This way, pictures (usually two-dimensional pictures; although line-like pictures, quasi-two-dimensional pictures or three-dimensional pictures are also possible) can be obtained. Using such images, a preferred method of monitoring the irradiation can usually be achieved. This is because typically images are very understandable to an operator of the irradiation arrangement. Furthermore, by using data from an imaging device, a broad scope of usable data can be gained that can be used for automated processing, like as an input for a computer or an electronic controller.

It is even more preferred, if the irradiation arrangement is designed in a way that said at least one position-sensitive particle monitoring device is designed, at least in part, as a time resolving monitoring device. This way, it is possible to achieve yet another piece of information. In particular, a time evolvement can be considered when performing the measurements. In particular in connection with an imaging device, it is possible to create a film, showing the temporal behavior of the workpiece that is monitored (and irradiated).

Furthermore, it is suggested to design the irradiation arrangement in a way that said irradiation arrangement, in particular said at least one particle beam supply device is designed for supplying particles to the workpiece to be irradiated, wherein said particles are taken from the group that comprises hadronic particles, charged particles, ions, mesons, protons, helium ions, heavy ions, oxygen ions, neon ions and carbon ions. Such particles have proven to be particularly suitable in connection with the suggested irradiation arrangement in first experiments. This might be contributable to the fact that those particles have a comparatively high mass (rest mass), so that they are not too much influenced by an interaction with the matter of the workpiece, so that they can still deliver usual information. It is to be noted that the use of hadronic particles in a way to penetrate the workpiece to be irradiated is very unusual. Usually, hadronic particles are used in a way that the particles "get stuck" in the workpiece, since the Bragg-peak is the very unique feature of hadronic particles that was thought to be employed on a quasi-mandatory level. Deviating from this concept is quite unusual.

Furthermore, it is suggested that the irradiation arrangement is designed in a way that the irradiation arrangement, in particular the at least one particle beam supply device is designed for supplying particles to the workpiece to be irradiated, wherein said particles have an energy of ≥ 400 MeV, preferably of ≥ 600 MeV more preferably of ≥ 800 MeV, even more preferred of ≥ 1000 MeV. First experiments have shown that such a very high energy of particles (in particular hadrons, protons, ions, helium ions) is particularly preferred. When such a high energy is employed, the particle beam penetrates the workpiece to be irradiated in a way that bending of individual particles you to interactions with the matter of the workpiece is still comparatively low, so that they can be detected in a distally located detector device and can still deliver useful information. Nevertheless, the interactions cause sufficiently large changes so that data can be derived from a measurement from distally located sensor devices.

A preferred embodiment of the irradiation arrangement can be achieved, if the irradiation arrangement is designed in a way that a dose of more than (and presumably including) 1 Gy, 5 Gy, 10 Gy, 25 Gy, 50 Gy or 100 Gy and/or a dose of more than (and presumably including) 1 Sv, 5 Sv, 10 Sv, 25 Sv, 50 Sv or 100 Sv is deposited in the workpiece to be treated. In particular, when talking about the unit "Gray" (Gy) the (usually more appropriate) units Gy (RBE) (which describes the deposited dose, including the relative biological effectivity "RBE") and GyE (which is the unit Gray Equivalent, typically the unit Cobalt Gray Equivalent) can be used additionally and/or alternatively as well. This has to be compared with doses that are applied to the workpiece, if a "pure" radiography is performed. In such a case, typically the applied doses are smaller than (and possibly equivalent to) 100 mGy, 50 mGy, 25 mGy, 10 mGy, 5 mGy, 2.5 mGy or 1 mGy and/or smaller than (and possibly equivalent to) 100 mSv, 50 mSv, 25 mSv, 10 mSv, 5 mSv, 2.5 mSv or 1 mSv.

Furthermore, it is suggested that the irradiation arrangement, in particular said at least one workpiece accommodation device is designed for accommodating animals and/or human beings and/or irradiation phantom devices and/or comprises at least one table and/or at least one stretcher. This way, the irradiation arrangement can be suitably used in the medical and/or veterinary field.

Furthermore it is suggested that in the irradiation arrangement, at least one particle beam supply device and/or at least one workpiece accommodation device are designed, at least in part, to be movable, in particular relative to each other. This way, a suitable entrance angle can be used for irradiating the workpiece. Furthermore, if the inner volume of the workpiece has to be irradiated, it is possible to perform the irradiation from different directions. This way, the dose that is deposited in the workpiece is comparatively low in areas outside of the inner volume to be irradiated, while the inner volume can be deposited with a high dose, if the irradiation is performed in a way that inner volume is transversed by the particle beams for a plurality/(essentially) all incident directions.

It is suggested to provide the irradiation arrangement with at least one additional distal particle monitoring device that is arranged, at least in part, on the distal side of said at least one workpiece accommodation device, wherein said at least one additional distal particle monitoring device is preferably designed as a particle charge monitoring device and/or as a particle energy monitoring device and/or as a particle type monitoring device. This way, additional data can be gained (apart from the at least one position-sensitive particle monitoring device). It is possible to use devices that perform a single measurement or to use devices that perform a plurality of measurements (for example a monitoring device that is measuring at least one position and the energy of the particle beam).

It is further suggested to provide the irradiation arrangement with at least one proximal particle monitoring device that is arranged, at least in part, on the proximal side of said at least one workpiece accommodation device, wherein said at least one proximal particle monitoring device is preferably designed as a position-sensitive particle monitoring device and/or as a particle charge monitoring device and/or as a particle energy monitoring device and/or as a particle type monitoring device. Using such an embodiment, it is possible to obtain data from the incident particle beam, before it has interacted with (parts of) the workpiece. This can be important for performing certain calculations. Furthermore it is possible to compare the data of the "ingoing" particle beam and the "outgoing" particle beam.

Another preferred embodiment can be obtained if the irradiation arrangement is equipped with at least one lateral particle monitoring device that is arranged, at least in part, on a lateral side of said at least one workpiece accommodation device, wherein said at least one lateral particle monitoring device is preferably designed as a position sensitive particle monitoring device and/or as a particle charge monitoring device and/or as a particle energy monitoring device and/or as a particle type monitoring device and/or as a photon detecting device. Using such an arrangement radiation that is emitted due to interactions of the particle beam with the matter of the workpiece can be measured as well, for example. Also, heavily scattered particles (in particular of the particle beam) can be measured as well. It can be particularly preferred to use a photon detecting device, since such a device can detect the so-called "prompt gammas", which are generated when an interaction between the particle beam and the matter of the workpiece takes place.

Yet another preferred embodiment is achieved, if the irradiation arrangement is equipped with at least one dose deposition determination system. It is possible to use a certain detector for this. Additionally or alternatively it is possible to use the data already present (for example supplied by the diverse monitoring devices) and to perform certain calculations based on this.

Yet another preferred embodiment of the irradiation arrangement can be achieved if at least one particle beam supply device of the irradiation arrangement comprises at least one particle accelerator device, in particular at least one linear accelerator device and/or at least one synchrotron device and/or at least one closed loop accelerator device and/or at least one laser induced accelerator device. Such accelerators have proven to be well-suited for generating a suitable particle beam for operating the irradiation arrangement.

It is preferred to design the irradiation arrangement in a way that the particle beam has a width, in particular a full width at half maximum (FWHM) width of ≥ 0.1 cm, preferably of ≤ 0.05 cm, more preferred of ≤ 0.01 cm, even more preferred of ≤ 0.005 cm. Using such a very thin particle beam, a very precise irradiation of the workpiece can be performed. In particular small structures within the workpiece to be irradiated can be precisely irradiated, when such a beam is used. However, other diameters are possible as well, for example a full width at half maximum (FWHM) width of ≤ 2 cm, preferably of ≤ 1.5 cm, more preferred of ≤ 1 cm, even more preferred of ≤ 0.5 cm. It might be even desirable to enlarge the beam diameter, using some kind of a beam spreading device. As an example, a large field irradiation with a diameter of up to 10 cm, 12 cm, 15 cm or even 20 cm might be possible. It should be mentioned that the beam (spreaded beam) does not necessarily have to have an approximately circular shape. Instead, other shapes, like essentially rectangular shapes, quadratic shapes or the like are possible as well.

Furthermore, a method for controlling an irradiation arrangement for irradiating a workpiece with a particle beam, in particular a method for controlling an irradiation arrangement according to the previous description is suggested, wherein a particle beam is used that is penetrating the workpiece to be irritated, and wherein the position of the particle beam, exiting the workpiece is monitored. Using such a method, the already described features and advantages of the irradiation arrangement can be realized for the method as well, at least in analogy. Furthermore the method can be modified in the previously described sense.

The invention will become clearer when considering the following description of embodiments of the present invention, together with the enclosed figures. The figures are showing:
- Fig. 1:: a high energy proton irradiation device in a schematic sketch;
- Fig. 2:: the diameter of a proton beam over the target thickness at several energy levels;
- Fig. 3:: the relative dose in dependence of the distance from the beam axis for a proton beam;
- Fig. 4:: the track-averaged linear energy transfer (LET) in air over the distance from the beam axis;
- Fig. 5:: model calculations for the diameter of a proton beam over the target thickness at several energy levels, using different theoretical models;
- Fig. 6:: a schematic sketch of a method for controlling an irradiation arrangement for irradiating a workpiece.

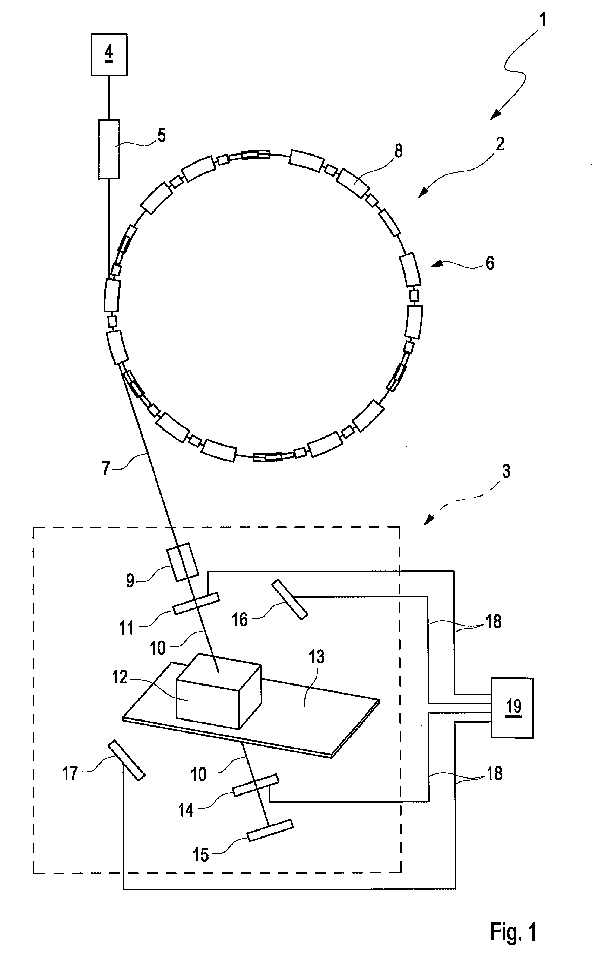

Fig. 1 shows a first possible embodiment of an irradiation device 1 in a schematic sketch. The irradiation device 1 can be divided into two main subgroups, mainly the accelerator section 2 and the irradiation room 3. In the presently shown example, the irradiation device 1 is used for medical purposes, so the irradiation room 3 would normally be addressed as a treatment room. The accelerator section 2 itself consists of three main units, namely the ion source 4, a linear accelerator 5 (often referred to as a LINAC) and a closed loop accelerator, in the present case a synchrotron 6. The linear accelerator 5 is used to initially accelerate the ions that are produced by the ion source 4. The thus accelerated ions 10 are then injected into a synchrotron 6, where they are accelerated in a closed loop and finally ejected to be transported via a transport pipe 7 to the irradiation room 3. The synchrotron 6 can be designed according to standard principles and usually shows a variety of various magnets 8 that are used for bending the ion beam (dipole magnets) and focusing the ion beam (quadropole magnets). Further, injector and extractor units (including kicker magnets) and accelerating parts are used (presently not shown).

In the presently shown example of an irradiation device 1, the ion source 4 is preferably designed to be a proton source (i.e. hydrogen ions). However, different types of ions are possible as well.

The irradiation room 3 comprises several units as well. The ion beam 10 that is guided to the irradiation room 3 via the transport pipe 7 is introduced into the irradiation room 3 via a differential window 9. Such a differential window 9 is necessary to be able to operate the irradiation room 3 at standard atmospheric conditions, while the inner parts of the accelerator section 2 are operated under vacuum (usually ultrahigh vacuum).

In the presently shown example, the ion beam 10 is first guided through a first sensor unit 11 that is arranged proximal to the workpiece to be irradiated, presently an irradiation phantom 12 that is used to verify a treatment plan. In principle, the irradiation phantom 12 could be replaced by a human patient or by any other object that has to be irradiated.

The irradiation phantom 12 is placed on a stretcher 13 that can be moved relatively to the ion beam 10. This movement is not only possible in lateral directions, but also with respect to an angle between the ion beam 10 and the irradiation phantom 12. Preferably, the stretcher 13 comprises some type of a fixture device (presently not shown), so that the workpiece to be irradiated (including irradiation phantoms 12 and patients) can be exactly positioned with respect to the ion beam 10.

In the presently shown example of an irradiation device 1, the ions (presently protons) are accelerated to so-called relativistic speeds. I.e., their energies are typically in the order of 800 MeV, 1 GeV and/or even higher. The reason for this will become clear with the following description.

Due to the high energy of the ions (protons), the ion beam 10 is penetrating ("going through") the irradiation phantom 12. Consequently, there is "something left over" for a second sensor unit 14 that is placed distal to the irradiation phantom 12. In the present case, the sensor unit 14 is designed as a time resolving imaging device. In particular, the imaging detector is fast enough to generate images at a sufficiently high sampling rate.

After the second sensor unit 14 (as seen in the direction of the ion beam 10), a beam dump 15 is located.

As can be seen from Fig. 1, in the presently shown embodiment a pair of two other sensor units 16, 17, namely a third sensor unit 16 and a fourth sensor unit 17 are employed. The third and fourth sensor unit 16, 17 are arranged "sideward" to the ion beam 10. Usually, third and fourth sensor unit 16, 17 are fast and amplifying photon detectors (in particular, a photomultiplier device can be used). Of course more sensor units can be used as well. Also, third and fourth sensor unit 16, 17 (and presumably even more sensor units) can be arranged to be movable. Third and fourth sensor unit 16, 17 are mainly used for detecting the so-called prompt gamma rays that are generated if an interaction between the ion beam 10 and matter of the irradiation phantom 12 occurs. All data collected by the different sensor units 11, 14, 16, 17 is fed via data lines 18 to an electronic computer 19, where appropriate calculations take place. In particular, the computer 19 can calculate a time resolving series of images (i.e. a "time-dependent" film). Furthermore, the data collected is usually sufficient to calculate the space-resolved dose that is actually deposited in the irradiation phantom 12 during irradiation. Such a calculation of the "really" deposited dose is particularly useful, if a patient is treated (instead of the presently shown irradiation phantom 12).

It is to be noted that the ion beam 10 used for "treatment" of the irradiation phantom 12 and for generating a presently time-evolving radiograph is the very same. It is not the case that different particles are mixed or that different beams from different directions or the like are used.

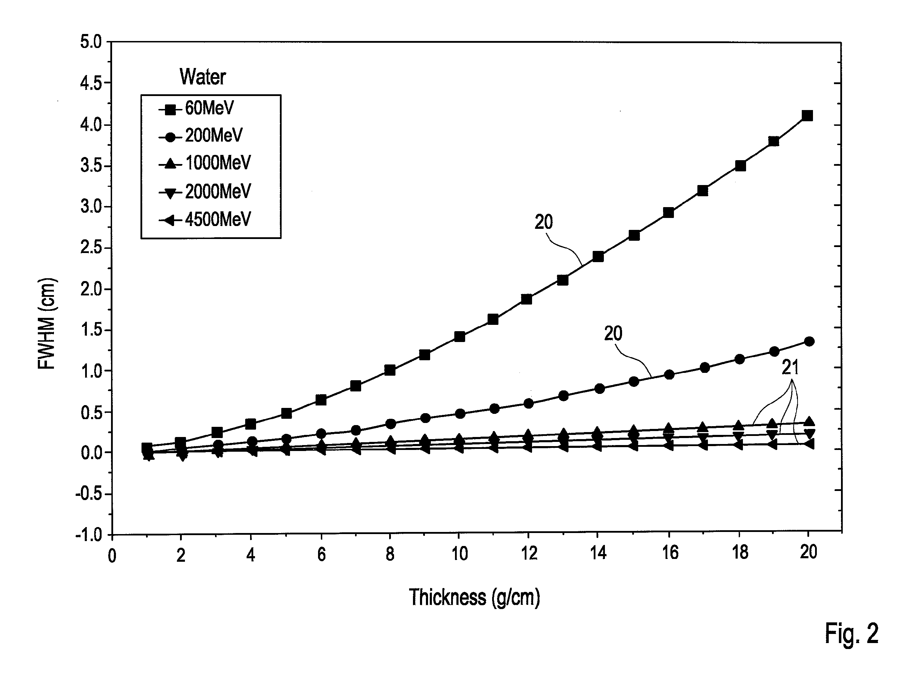

The advantage of using relativistic ions (in particular relativistic protons) is that they are influenced only insignificantly, in particular when compared to slow protons. An example for this can be seen in Fig. 2. In the presently employed state of the art, protons with energies of typically up to 60 MeV (and in some cases even up to 200 MeV) are generally employed. It can be seen from Fig. 2, that the FWHM diameters for slow protons (see FWHM lines 20) are rapidly increasing with the target thickness. FWHM stands for full width at half maximum. This is actually the reason, why ions have not been used for producing radiographs, so far (if they were used in a way to penetrate the irradiation phantom 12 at all). Due to the broadening of the ion beam 10 , the ion beam 10 would not be fine enough for medical applications. Furthermore, measurements on the distal side of the irradiation phantom 12 would be too disturbed to be usable.

The situation is a different, if relativistic ions (protons) are used. This can be clearly seen from the FWHM lines 21 for relativistic protons.

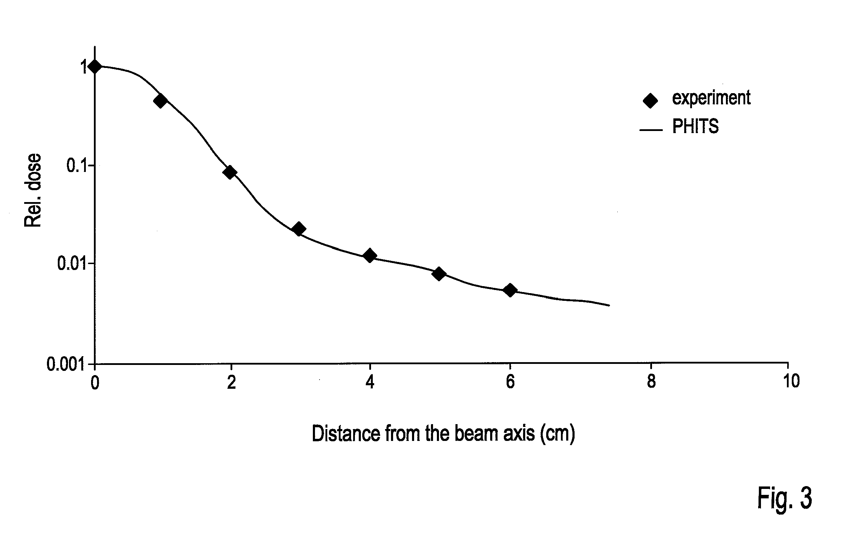

Another advantageous feature when using relativistic ions (protons) can be seen in Fig. 3. Here, the relative dose is plotted over the distance from the beam axis. The plot is indicating a proton beam with an incident energy of 1 GeV for the protons. As can be seen, the deposited dose is well located in the middle of the beam. Please note the logarithmic scale for the relative dose.

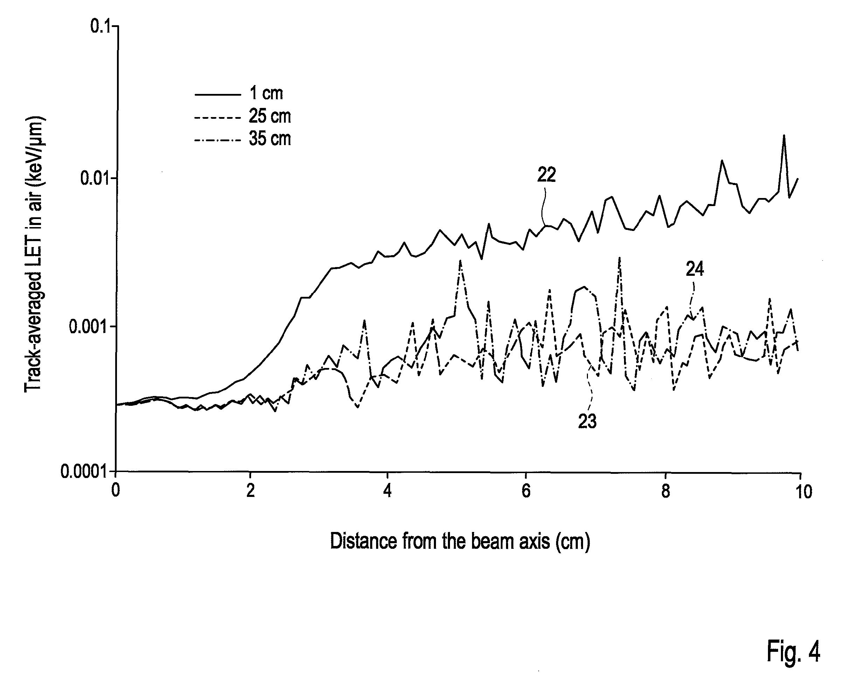

In Fig. 4 the track-averaged LET in air is plotted over the distance from the beam axis for different penetration depths, namely at a depth of 1 cm 22, at a depth of 25 cm 23 and at a depth of 35 cm 24. LET is the commonly used abbreviation for linear energy transfer rate.

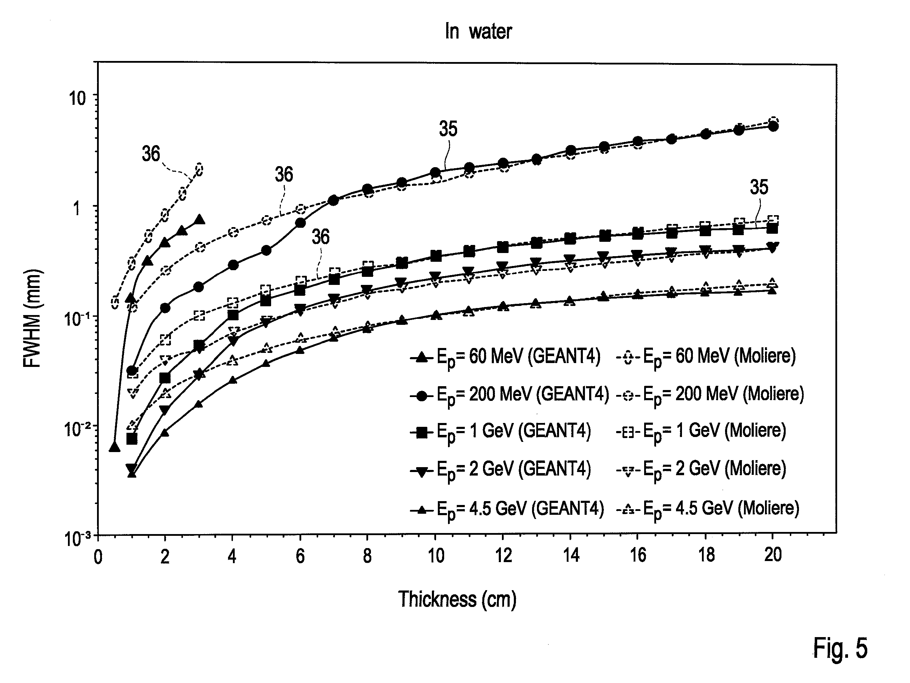

Fig. 5 is somewhat similar to the already described Fig. 2. Similar to Fig. 2, present Fig. 5 shows the FWHM diameters for a proton beam at different incident energy levels over the penetrated length in the irradiated object. The data shown is based on calculations, where two different theoretical models (computer codes) have been used, namely the GEANT4-code, as well as the Moliere-code. Both are frequently used in research. The solid lines 35 (GEANT-lines 35) in Fig. 5 are based on calculations, performed with the GEANT4-code, while the dashed lines 36 (Moliere-lines 36) are based on calculations, performed with the Moliere-code. As can be seen from Fig. 5, a significant improvement in FWHM-width can be realised, if protons in the relativistic energy regime are used (in Fig. 5 energies of 1 GeV, 2 GeV and 4.5 GeV), when compared to comparatively slow protons (in Fig. 5 energies of 60 MeV and 200 MeV). When comparing the different lines 35, 36, one has to keep in mind the logarithmic scale of the ordinate in Fig. 5. As one can also see from Fig. 5, the different computer codes yield somewhat different results; nevertheless, it is clearly indicated that the use of protons with relativistic energies results in a major reduction of the proton beam diameter behind the irradiated object.

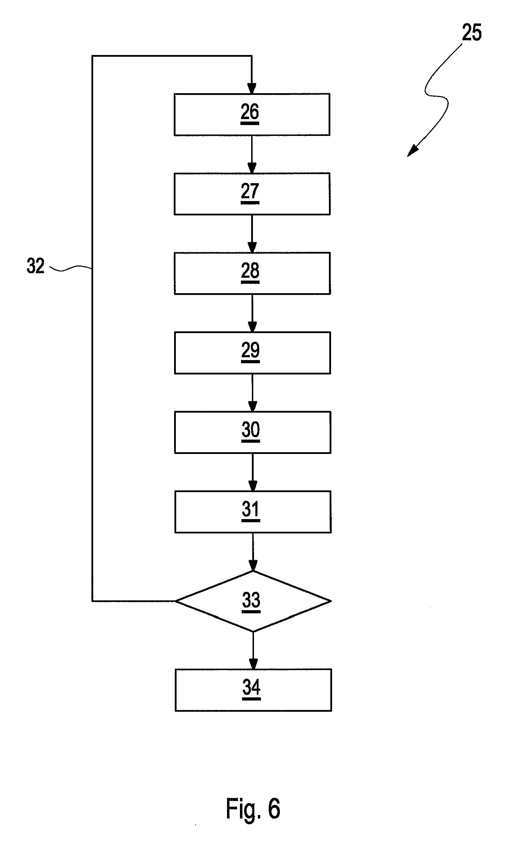

In Fig. 6, a schematic flowchart 25 is illustrating a possible embodiment for a method of controlling an irradiation device (for example the irradiation device 1 of Fig. 1). Of course it is possible that the method 25 is running on a computer 19 that is used for measurement purposes as well. In this case, additional data lines 18 have to be used between the computer 19 and the acceleration devices. Usually, however, the accelerator section 2 is controlled by separate computers. The method 25 starts with providing an ion beam 26 with the desired parameters. Then the beam parameters are measured 27 on the proximal side of the workpiece 12 to be irradiated. Likewise, the beam parameters are measured 28 on the distal side of the workpiece 12 to be irradiated as well. Furthermore measurements take place 29 on lateral sides of the workpiece 12 to be irradiated as well (in particular using photo detectors for detecting so-called fast gamma rays) .

Now a radiograph and the "really" deposited dose is calculated in a calculation step 30. Depending on the result of the calculation at step 30 the irradiation then that is still to be applied can be modified 31, if necessary. Although a radiograph can be generated and outputted, even while the irradiation still takes place (additionally and/or alternatively), one can also wait to the very end of the irradiation process.

Now the method jumps back 32 if the irradiation is not yet finished. However, if in a checking step 33 it is determined that the irradiation is completed, the method will stop 34.

**Reference list:**

| | | | |
|---|---|---|---|
| 1. | Irradiation device | 20. | FWHM line (slow protons) |
| 2. | accelerator section | 21. | FWHM line (relativistic pro-tons) |
| 3. | Irradiation room | | |
| 4. | Ion source | 22. | 1 cm depth line |
| 5. | Linear accelerator | 23. | 25 cm depth line |
| 6. | Synchrotron | 24. | 35 cm depth line |
| 7. | Transport pipe | 25. | flowchart |
| 8. | Magnet | 26. | Providing ion beam |
| 9. | Differential window | 27. | Proximal measurements |
| 10. | Ion beam | 28. | Distal measurements |
| 11. | First sensor unit | 29. | Lateral measurements |
| 12. | Irradiation phantom | 30. | Calculation step |
| 13. | Stretcher | 31. | Alternation of irradiation |
| 14. | Sensor unit | 32. | Jump back |
| 15. | Beam dump | 33. | Check step |
| 16. | Third sensor unit | 34. | Stop |
| 17. | Fourth sensor unit | 35. | GEANT-line |
| 18. | Data line | 36. | Moliere-line |
| 19. | Computer | | |

## Claims

1. Irradiation arrangement (1, 3) for irradiating a workpiece (12) with a particle beam (10) of particles having a rest mass, comprising a particle beam supply device (2, 9), at least one workpiece accommodation device (13) and at least one position-sensitive particle monitoring device (14), wherein said at least one position-sensitive particle monitoring device (14) is arranged at least in part on the distal side of said at least one workpiece accommodation device (13), when seen from said at least one particle beam supply device (2, 9), **characterised in that** said particle beam supply device (2, 9) delivers a single particle beam (10) that is used to irradiate the workpiece (12) for treatment purposes and that is detected by said position-sensitive particle monitoring device (14) for monitoring purposes.

2. Irradiation arrangement (1, 3) according to claim 1, **characterised in that** said at least one position-sensitive particle monitoring device (14) is designed, at least in part, as an imaging device.

3. Irradiation arrangement (1, 3) according to claim 1 or 2, in particular according to claim 2, **characterised in that** said at least one position-sensitive particle monitoring device (14) is designed, at least in part, as a time resolving monitoring device.

4. Irradiation arrangement (1, 3) according to any of the preceding claims, **characterised in that** said irradiation arrangement (1, 3), in particular said at least one particle beam supply device (2, 9) is designed for supplying particles (10) to the workpiece (12) to be irradiated, wherein said particles (10) are taken from the group that comprises hadronic particles, charged particles, ions, mesons, protons, helium ions, heavy ions, oxygen ions, neon ions and carbon ions.

5. Irradiation arrangement (1, 3) according to any of the preceding claims, in particular according to claim 4, **characterised in that** said irradiation arrangement (1, 3), in particular said at least one particle beam supply device (2, 9) is designed for supplying particles (10) to the workpiece (12) to be irradiated, wherein said particles (10) have an energy of ≥ 400 MeV, preferably of ≥ 600 MeV, more preferably of ≥ 800 MeV, even more preferred of ≥ 1000 MeV.

6. Irradiation arrangement (1, 3) according to any of the preceding claims, **characterised in that** said irradiation arrangement (1, 3), in particular said at least one workpiece accommodation device (13) is designed for accommodating animals and/or human beings and/or irradiation phantom devices (12) and/or comprises at least one table and/or at least one stretcher (13).

7. Irradiation arrangement (1, 3) according to any of the preceding claims, **characterised in that** said at least one particle beam supply device (2, 9) and/or said at least one workpiece accommodation device (13) are designed, at least in part, to be movable, in particular relative to each other.

8. Irradiation arrangement (1, 3) according to any of the preceding claims, **characterised by** at least one additional distal particle monitoring device that is arranged, at least in part, on the distal side of said at least one workpiece accommodation device (13), wherein said at least one additional distal particle monitoring device is preferably designed as a particle charge monitoring device and/or as a particle energy monitoring device and/or as a particle type monitoring device.

9. Irradiation arrangement (1, 3) according to any of the preceding claims, **characterised by** at least one proximal particle monitoring device (11) that is arranged, at least in part, on the proximal side of said at least one workpiece accommodation device (13), wherein said at least one proximal particle monitoring device (11) is preferably designed as a position-sensitive particle monitoring device and/or as a particle charge monitoring device and/or as a particle energy monitoring device and/or as a particle type monitoring device.

10. Irradiation arrangement (1, 3) according to any of the preceding claims, **characterised by** at least one lateral particle monitoring device (16, 17) that is arranged, at least in part, on a lateral side of said at least one workpiece accommodation device (13), wherein said at least one lateral particle monitoring device (16, 17) is preferably designed as a position-sensitive particle monitoring device and/or as a particle charge monitoring device and/or as a particle energy monitoring device and/or as a particle type monitoring device and/or as a photon detecting device.

11. Irradiation arrangement (1, 3) according to any of the preceding claims, in particular according to any of claims 8 to 10, **characterised by** at least one dose deposition determination system (11, 14, 16, 17, 19).

12. Irradiation arrangement (1, 3) according to any of the preceding claims, **characterised in that** said at least one particle beam supply device comprises (2, 9) at least one particle accelerator device (2, 5, 6), in particular at least one linear accelerator device (5) and/or at least one synchrotron device (6) and/or at least one closed loop accelerator device and/or at least one laser induced accelerator device.

13. Irradiation arrangement (1, 3) according to any of the preceding claims, in particular according to claim 12, **characterised in that** said particle beam has a width, in particular a full width at half maximum (FWHM) width of ≤ 0.1 cm, preferably of ≤ 0.05 cm, more preferred of ≤ 0.01 cm, even more preferred of ≤ 0.005 cm.

14. Method (25) for controlling an irradiation arrangement for irradiating a workpiece (12) with a particle beam (10), in particular method (25) for controlling an irradiation arrangement (1, 3) according to any of claims 1 to 13, wherein a particle beam (10) is used that is penetrating the workpiece (12) to be irradiated, **characterised in that** the position of the particle beam (10), exiting the workpiece (12) is monitored (28).

## Patentansprüche

1. Bestrahlungsanlage (1, 3) für die Bestrahlung eines Werkstücks (12) mit einem Teilchenstrahl (10), wobei diese Teilchen eine Ruhemasse haben, umfassend einen Teilchenstrahlerzeuger (2, 9), mindestens eine Werkstückhalterung (13) und mindestens eine positionsempfindliche Teilchenüberwachungsvorrichtung (14), wobei diese mindestens eine positionsempfindliche Teilchenüberwachungsvorrichtung (14) zumindest teilweise auf der aus Sicht des mindestens einen Teilchenstrahlerzeugers (2, 9) distal der mindestens einen Werkstückhalterung (13) angeordnet ist, **dadurch gekennzeichnet, dass** der besagte Teilchenstrahlerzeuger (2, 9) einen einzelnen Teilchenstrahl abgibt, der für die Bestrahlung eines Werkstücks (12) zu Behandlungszwecken benutzt wird und der von der besagten positionsempfindlichen Teilchenüberwachungsvorrichtung (14) zu Überwachungszwecken erkannt wird.

2. Bestrahlungsanlage (1, 3) nach Anspruch 1, **dadurch gekennzeichnet, dass** die mindestens eine positionsempfindliche Teilchenüberwachungsvorrichtung (14) mindestens teilweise als bildgebende Vorrichtung konzipiert ist.

3. Bestrahlungsanlage (1, 3) nach Anspruch 1 oder 2, besonders nach Anspruch 2, **dadurch gekennzeichnet, dass** die mindestens eine positionsempfindliche Teilchenüberwachungsvorrichtung (14) zumindest teilweise als zeitauflösende Überwachungsvorrichtung konzipiert ist.

4. Bestrahlungsanlage (1, 3) nach einem der vorstehenden Ansprüche, **dadurch gekennzeichnet, dass** die Bestrahlungsanlage (1, 3), insbesondere der mindestens eine Teilchenstrahlerzeuger (2, 9), so konzipiert ist, um das zu bestrahlende Werkstück (12) mit Teilchen (10) zu bestrahlen, wobei die besagten Teilchen (10) aus der Gruppe ausgewählt werden, die Hadronen-Teilchen, geladene Teilchen, Ionen, Mesonen, Protonen, Helium-lonen, schwere Ionen, Sauerstoff-Ionen, Neon-Ionen und Kohlenstoff-Ionen umfasst.

5. Bestrahlungsanlage (1, 3) nach einem der vorstehenden Ansprüche, besonders nach Anspruch 4, **dadurch gekennzeichnet, dass** die Bestrahlungsanlage (1, 3), insbesondere der mindestens eine Teilchenstrahlerzeuger (2, 9), so konzipiert ist, um das zu bestrahlende Werkstück (12) mit Teilchen (10) zu bestrahlen, wobei die besagten Teilchen (10) eine Energie von ≥ 400 MeV, vorzugsweise von ≥ 600 MeV, noch besser von ≥ 800 MeV und im besten Fall von ≥ 1000 MeV aufweisen.

6. Bestrahlungsanlage (1, 3) nach einem der vorstehenden Ansprüche, **dadurch gekennzeichnet, dass** die Bestrahlungsanlage (1, 3), insbesondere die mindestens eine Werkstückhalterung (13), so konzipiert ist, dass darauf Tiere und/oder Menschen und/oder Bestrahlungsphantomvorrichtungen (12) gelagert werden können und/oder dass sie mindestens einen Tisch und/oder eine Tragbahre (13) umfasst.

7. Bestrahlungsanlage (1, 3) nach einem der vorstehenden Ansprüche, **dadurch gekennzeichnet, dass** der mindestens eine Teilchenstrahlerzeuger (2, 9) und/oder die mindestens eine Werkstückhalterung (13), zumindest teilweise so konzipiert sind, dass sie, insbesondere im Verhältnis zueinander, beweglich sind.

8. Bestrahlungsanlage (1, 3) nach einem der vorstehenden Ansprüche, **dadurch gekennzeichnet, dass** mindestens eine zusätzliche distale Teilchenüberwachungsvorrichtung zumindest teilweise auf der distalen Seite der mindestens einen Werkstückhalterung (13) angeordnet ist, wobei diese mindestens eine zusätzliche distale Teilchenüberwachungsvorrichtung vorzugsweise als Überwachungsvorrichtung der Teilchenladung und/oder als Überwachungsvorrichtung der Teilchenenergie und/oder als Überwachungsvorrichtung der Teilchenart konzipiert ist.

9. Bestrahlungsanlage (1, 3) nach einem der vorstehenden Ansprüche, **dadurch gekennzeichnet, dass** mindestens eine proximale Teilchenüberwachungsvorrichtung (11) zumindest teilweise auf der proximalen Seite der mindestens einen Werkstückhalterung (13) angeordnet ist, wobei diese mindestens eine proximale Teilchenüberwachungsvorrichtung (11) vorzugsweise als positionsempfindliche Teilchenüberwachungsvorrichtung und/oder als Überwachungsvorrichtung der Teilchenladung und/oder als Überwachungsvorrichtung der Teilchenenergie und/oder als Überwachungsvorrichtung der Teilchenart konzipiert ist.

10. Bestrahlungsanlage (1, 3) nach einem der vorstehenden Ansprüche, **dadurch gekennzeichnet, dass** mindestens eine seitliche Teilchenüberwachungsvorrichtung (16, 17) zumindest teilweise seitlich neben der mindestens einen Werkstückhalterung (13) angeordnet ist, wobei diese mindestens eine seitliche Teilchenüberwachungsvorrichtung (16, 17) vorzugsweise als positionsempfindliche Teilchenüberwachungsvorrichtung und/oder als Überwachungsvorrichtung der Teilchenladung und/oder als Überwachungsvorrichtung der Teilchenenergie und/oder als Überwachungsvorrichtung der Teilchenart und/oder als Photonen-Erkennungsvorrichtung konzipiert ist.

11. Bestrahlungsanlage (1, 3) nach einem der vorstehenden Ansprüche, besonders nach einem der Ansprüche 8 bis 10, **dadurch gekennzeichnet, dass** sie mindestens ein Ermittlungssystem für den Dosis-Eintrag (11, 14, 16, 17, 19) umfasst.

12. Bestrahlungsanlage (1, 3) nach einem der vorstehenden Ansprüche, **dadurch gekennzeichnet, dass** der mindestens eine Teilchenstrahlerzeuger (2, 9) mindestens einen Teilchenbeschleuniger (2, 5, 6) umfasst, insbesondere mindestens einen Linearbeschleuniger (5) und/oder mindestens ein Synchrotron (6) und/oder mindestens einen geschlossenen Ringbeschleuniger und/oder mindestens einen durch Laser angeregten Beschleuniger.

13. Bestrahlungsanlage (1, 3) nach einem der vorstehenden Ansprüche, besonders nach Anspruch 12, **dadurch gekennzeichnet, dass** der Teilchenstrahl eine Breite, insbesondere eine Halbwertsbreite (FWHM), von ≤ 0,1 cm, vorzugsweise von ≤ 0,05 cm, noch besser von ≤ 0,01 cm und im besten Fall von ≤ 0,005 cm aufweist.

14. Verfahren (25) zur Steuerung einer Bestrahlungsanlage zur Bestrahlung eines Werkstücks (12) mit einem Teilchenstrahl (10), insbesondere Verfahren (25) zur Steuerung einer Bestrahlungsanlage (1, 3) nach einem der Ansprüche 1 bis 13, wobei ein Teilchenstrahl (10) verwendet wird, der das zu bestrahlende Werkstück (12) durchdringt, **dadurch gekennzeichnet, dass** die Position des Teilchenstrahls (10) am Austritt aus dem Werkstück (12) überwacht wird (28).

## Revendications

1. Installation de rayonnement (1, 3) conçue pour exposer une pièce de travail (12) au rayonnement d'un faisceau de particules (10), les particules ayant une masse au repos, et comprenant un dispositif d'émission de faisceau de particules (2, 9), au moins un dispositif de réception de pièce (13) et au moins un dispositif de surveillance de particules sensible à la position (14), ledit au moins un dispositif de surveillance de particules sensible à la position (14) étant au moins en partie disposé sur le côté distal dudit au moins un dispositif de réception de pièce (13), tel qu'observé depuis ledit au moins un dispositif d'émission de faisceau de particules (2, 9), **caractérisé en ce que** ledit dispositif d'émission de faisceau de particules (2, 9) délivre un seul faisceau de particules (10) qui est utilisé pour exposer la pièce (12) à un rayonnement à des fins de traitement et qui est détecté par ledit dispositif de surveillance de particules sensible à la position (14) à des fins de surveillance.

2. Installation de rayonnement (1, 3) selon la revendication 1, **caractérisée en ce que** ledit au moins un dispositif de surveillance de particules sensible à la position (14) est au moins en partie conçu comme un dispositif d'imagerie.

3. Installation de rayonnement (1, 3) selon la revendication 1 ou 2, en particulier selon la revendication 2, **caractérisée en ce que** ledit au moins un dispositif de surveillance de particules sensible à la position (14) est au moins en partie conçu comme un dispositif de surveillance à résolution temporelle.

4. Installation de rayonnement (1, 3) selon l'une quelconque des précédentes revendications, **caractérisée en ce que** ladite installation de rayonnement (1, 3), en particulier ledit au moins un dispositif d'émission de faisceau de particules (2, 9), est conçue pour émettre des particules (10) vers la pièce (12) devant être exposée au rayonnement, lesdites particules (10) étant sélectionnées dans le groupe comprenant des particules hadroniques, des particules chargées, des ions, des mésons, des protons, des ions hélium, des ions lourds, des ions oxygène, des ions néon et des ions carbone.

5. Installation de rayonnement (1, 3) selon l'une quelconque des précédentes revendications, en particulier selon la revendication 4, **caractérisée en ce que** ladite installation de rayonnement (1, 3), en particulier ledit au moins un dispositif d'émission de faisceau de particules (2, 9), est conçue pour émettre des particules (10) vers la pièce (12) devant être exposée au rayonnement, lesdites particules (10) ayant une énergie ≥ 400 MeV, de préférence ≥ 600 MeV, mieux encore ≥ 800 MeV et, dans l'idéal, ≥ 1000 MeV.

6. Installation de rayonnement (1, 3) selon l'une quelconque des précédentes revendications, **caractérisée en ce que** ladite installation de rayonnement (1, 3), en particulier ledit au moins un dispositif de réception de pièce (13), est conçue pour recevoir des animaux et/ou des êtres humains et/ou des dispositifs fantômes de rayonnement (12), et/ou comprend au moins une table et/ou au moins une civière (13).

7. Installation de rayonnement (1, 3) selon l'une quelconque des précédentes revendications, **caractérisée en ce que** ledit au moins un dispositif d'émission de faisceau de particules (2, 9) et/ou ledit au moins un dispositif de réception de pièce (13) sont au moins en partie conçus de manière à être mobiles, en particulier l'un par rapport à l'autre.

8. Installation de rayonnement (1, 3) selon l'une quelconque des précédentes revendications, **caractérisée par** au moins un dispositif de surveillance de particules distal supplémentaire qui est au moins en partie disposé sur le côté distal dudit au moins un dispositif de réception de pièce (13), ledit au moins un dispositif de surveillance de particules distal supplémentaire étant de préférence conçu comme un dispositif de surveillance de charge de particules et/ou comme un dispositif de surveillance d'énergie de particules et/ou comme un dispositif de surveillance de type de particules.

9. Installation de rayonnement (1, 3) selon l'une quelconque des précédentes revendications, **caractérisée par** au moins un dispositif de surveillance de particules proximal (11) qui est au moins en partie disposé sur le côté proximal dudit au moins un dispositif de réception de pièce (13), ledit au moins un dispositif de surveillance de particules proximal (11) étant de préférence conçu comme un dispositif de surveillance de particules sensible à la position et/ou comme un dispositif de surveillance de charge de particules et/ou comme un dispositif de surveillance d'énergie de particules et/ou comme un dispositif de surveillance de type de particules.

10. Installation de rayonnement (1, 3) selon l'une quelconque des précédentes revendications, **caractérisée par** au moins un dispositif de surveillance de particules latéral (16, 17) qui est au moins en partie disposé sur un côté latéral dudit au moins un dispositif de réception de pièce (13), ledit au moins un dispositif de surveillance de particules latéral (16, 17) étant de préférence conçu comme un dispositif de surveillance de particules sensible à la position et/ou comme un dispositif de surveillance de charge de particules et/ou comme un dispositif de surveillance d'énergie de particules et/ou comme un dispositif de surveillance de type de particules et/ou comme un dispositif de détection de photons.

11. Installation de rayonnement (1, 3) selon l'une quelconque des précédentes revendications, en particulier selon l'une quelconque des revendications 8 à 10, **caractérisée en ce qu'**elle comprend au moins un système de détermination de dépôt de dose (11, 14, 16, 17, 19).

12. Installation de rayonnement (1, 3) selon l'une quelconque des précédentes revendications, **caractérisée en ce que** ledit au moins un dispositif d'émission de faisceau de particules (2, 9) comprend au moins un dispositif accélérateur de particules (2, 5, 6), en particulier au moins un dispositif accélérateur linéaire (5) et/ou au moins un dispositif synchrotron (6) et/ou au moins un dispositif accélérateur en boucle fermée et/ou au moins un dispositif accélérateur excité par laser.

13. Installation de rayonnement (1, 3) selon l'une quelconque des précédentes revendications, en particulier selon la revendication 12, **caractérisée en ce que** ledit faisceau de particules a une largeur, en particulier une largeur à mi-hauteur (FWHM) ≤ 0,1 cm, de préférence ≤ 0,05 cm, mieux encore ≤ 0,01 cm et, dans l'idéal, ≤ 0,005 cm.

14. Procédé (25) pour piloter une installation de rayonnement conçue pour exposer une pièce (12) au rayonnement d'un faisceau de particules (10), en particulier procédé (25) pour piloter une installation de rayonnement (1, 3) selon l'une quelconque des revendications 1 à 13, dans lequel on utilise un faisceau de particules (10) qui pénètre dans la pièce (12) devant être exposée au rayonnement, **caractérisé en ce que** la position du faisceau de particules (10) à la sortie de la pièce (12) est surveillée (28).
